# EUROPEAN PATENT SPECIFICATION

(11) **EP 4 398 982 B1**
(45) Date of publication and mention of the grant of the patent: **15.10.2025**
(21) Application number: 22738734.7
(22) Date of filing: 17.06.2022
(51) Int. Cl.: A61N 2/02, A61N 1/40

(54) **FEEDBACK-CONTROLLED DEVICE FOR GEOMAGNETIC ION CYCLOTRON RESONANCE**
FEEDBACK-GESTEUERTE VORRICHTUNG FÜR GEOMAGNETISCHE IONENZYKLOTRONRESONANZ
DISPOSITIF COMMANDÉ PAR RÉTROACTION POUR LA RÉSONANCE CYCLOTRON IONIQUE GÉOMAGNÉTIQUE

(30) Priority: 18.06.2021 IT 202100015971
(43) Date of publication of application: 17.07.2024
(73) Proprietor: Non Ionizing Radiation Personal Shielding S.r.l., 53036 Poggibonsi (Siena) (IT)
(72) Inventor: GIULIANI, Livio, 00144 Roma (IT)
(74) Representative: Grassi, Stefano
(86) International application number: PCT/IB2022/055646
(87) International publication number: WO 2022/264104

(56) References cited:
- WO-A1-00/07664
- WO-A1-2007/077532
- WO-A1-2010/025114
- WO-A1-2017/194693
- WO-A1-93/12835
- WO-A1-95/31939
- WO-A2-2019/155407
- CN-A- 108 339 200
- US-A- 5 480 373

## Description

### Technical field

The present invention relates to the technical field of devices for healthcare use.

In particular, the present invention relates to a device for ion cyclotron resonance feedback-controlled by a variation in specific body impedance which also takes account of the real contribution/interference generated by the geomagnetic field.

### Prior art

Ion cyclotron resonance has been recognised as an interaction between low-frequency electromagnetic fields and living matter following the reproduction in various countries of the so-called BLZ (Blackman-Liboff-Zhadin) effect.

In particular, an important biological reaction has been recognised with reference to the action of very low-frequency electromagnetic fields on capillary and transmembrane ion motion.

The application of this physicochemical effect to biology has led to surprising results in regenerative medicine through the stimulation of metabolism and cell differentiation and maturation in neuronal and pseudo-neuronal cells, normal or cancerous epithelial cells, cardiac cells, and also in bone and cartilage cells.

Ion cyclotron resonance appears to play an important role also in the prevention and treatment of degenerative diseases such as sporadic and familial Alzheimer's disease, as it shows an ability to reduce β-amyloid plaques in the brain and also an antibacterial action, making it possible to modulate biofilm production by antibiotic-resistant bacteria.

However, the known devices are still affected by defects and disadvantages that preclude an efficient use of this technology, despite the enormous nascent interest in the countless possible applications in the medical field.

The main problem with the known devices is that they are not generally built to exploit a known bioelectromagnetic interaction, but are rather built on an empirical basis, and are generally closed systems, with a pre-set list of operating conditions that is presented to the therapist, who can thus not freely choose the frequencies, waveforms or intensities to be applied, but rather chooses a set of pre-set values that in his or her opinion are the best suited to the symptoms determined in the patient.

Even the most advanced devices, such as those mentioned for the treatment of tumours, do not allow the individual operating parameters to be analysed and selected precisely and accurately in order to optimise the therapeutic effect.

Furthermore, the known systems are not even configured to really operate under a condition of ion cyclotron resonance, as they are calibrated, designed and controlled on the basis of a fixed value for the geomagnetic field, thus without taking account of the variations therein, as is described, for example, in document WO2007/077532.

Given such imprecision, it is not actually possible to determine, in practice, any Liboff-Zhadin effect for any ion, thus making ion cyclotron resonance in fact inapplicable in the known devices.

### Object of the invention

In this context, the technical task at the basis of the present invention is to propose a feedback-controlled device for ion cyclotron resonance that overcomes at least some of the aforementioned drawbacks of the prior art. In particular, it is an object of the present invention to provide a feedback-controlled device for ion cyclotron resonance that can be easily and efficiently configured to work optimally according to the specific situation in which it operates, in particular according to the ambient conditions and the conditions of the patient undergoing the ion resonance process.

The stated technical task and specified objects are substantially achieved by a feedback-controlled device for ion cyclotron resonance comprising the technical features disclosed in one or more of the appended claims, which define the invention.

According to the present invention, there is shown a feedback-controlled device for ion cyclotron resonance which comprises an exposure system, a signal generator, an impedance meter and at least one gaussmeter.

The exposure system comprises a plurality of coils delimiting a treatment volume inside which a user can be at least partially accommodated and configured to generate an electromagnetic field inside the treatment volume;
The impedance meter is configured to record a specific impedance signal (i.e. one for each single frequency) representative of the impedance value at the extremities of the user's body when exposed to the electromagnetic field inside the treatment volume; this value depends on the frequency administered at that moment.

The gaussmeter is configured to generate a reference signal representative of an intensity value of a spatial distribution of the electromagnetic field inside the treatment volume and calculated also as a function of the magnetic inclination of that field.

The signal generator is configured to control the coils by modulating one or more characteristics of the electromagnetic field as a function, simultaneously, of the impedance signal and the reference signal, generating an operating signal at frequencies comprised between 100 and 300 kHz.

Advantageously, the device according to the present invention makes it possible to carry out a ion resonance treatment in an optimal, particularly efficient manner thanks to the real-time control of the electromagnetic field enabled by the continuous monitoring of the field distribution thereof and of the user's response to treatment through an assessment of his or her specific impedance at the various frequencies that can be delivered by the signal generator.

The dependent claims correspond to different embodiments of the invention.

### Brief description of the drawings

Additional features and advantages of the present invention will become more apparent from the description of a preferred embodiment of a feedback-controlled device for ion cyclotron resonance, as illustrated in the accompanying drawings, in which:

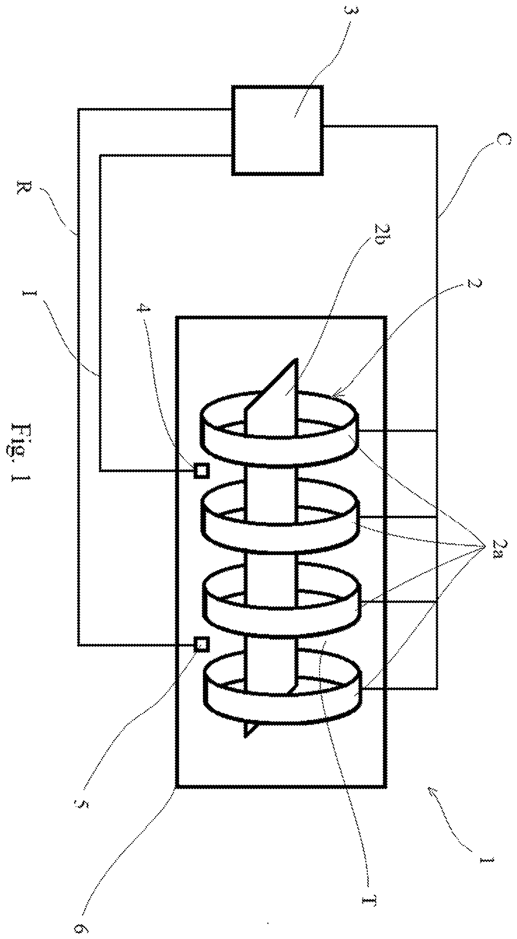

- figure 1 shows a diagram representative of the main constituent elements of the device according to the present invention;

### Detailed description of preferred embodiments of the invention

In the appended figures, the reference number 1 generically denotes a feedback-controlled device for ion cyclotron resonance, also identifiable and definable with the term "ion cyclotron", which will be referred to hereinafter in the present description simply as the device 1.

More specifically, the device described herein is a device for the ion cyclotron resonance feedback-controlled by a variation in specific body impedance.

Ion cyclotron resonance is a phenomenon correlated with the movement of ions in a magnetic field and of a voltage signal delivered at an appropriate frequency.

Under the action of the magnetic field and the applied voltage the charged particles are accelerated according to a spiral motion.

Ion cyclotron resonance is not limited to the induction of cellular ionic currents of inorganic ions, but can be applied in the healthcare realm as well, since it can also be applied to organic molecules and is founded on a precise mechanism, whereby it is possible to induce in cells, also in vivo, ionic currents also originating from heavy ions and zwitterions, which exploit, for the formation and persistence thereof, the energy released by coherent clusters of water molecules present in cells.

These clusters, called coherence domains, can attract ions and release their electromagnetic energy so as to generate ionic currents in the solution, which have sufficient energy to overcome the potential barrier, as occurs for example in the Liboff-Zhadin effect.

Structurally, the device 1 comprises an exposure system 2, a signal generator 3, an impedance meter 4, at least one gaussmeter 5 and a feedback circuit.

The exposure system 2 comprises a plurality of coils 2a, delimiting a treatment volume "T", in which a user can be at least partially accommodated, and configured to administer an electromagnetic field inside the treatment volume "T"

In particular, the coils 2a have respective inner surfaces that combine to define the treatment volume "T" adapted to at least partially accommodate the user therewithin.

In other words, the coils 2a have a substantially annular shape and dimensions such that their inside diameter is sufficiently large to permit a user to position him- or herself inside them; hence, the inner surface of the coils forms a portion of the wall of the treatment volume "T".

In accordance with a first possible embodiment, the support 2b comprises a bed for the user, which the latter may lie upon during use of the device 1.

It may be observed that the term bed means any type of support on which the user may rest in a lying position, for example a hospital bed.

In this context, the plurality of coils 2a comprises a plurality of Helmholtz coils, preferably four Helmholtz coils.

In general, Helmholtz coils consist of identical circular magnetic coils that are placed symmetrically along a common axis and spaced equally apart so as to be evenly distributed relative to the treatment volume "T" they delimit.

Preferably, the Helmholtz coils are separated by a distance equal to the radius of the coils themselves; in this manner, the non-uniformity of the electromagnetic field at the centre of the coils is minimised.

Preferably, there is a first Helmholtz coil associated with the head end of the bed and a second Helmholtz coil associated with the foot end thereof, with a further two Helmholtz coils optionally in an intermediate position and preferably movable axially (for example by means of a motor) between a non-operating position, in which they are brought alongside the first or second coil, respectively, so as to enable the user to lie down on the bed, and an operating position in which all coils are spaced equally apart from the adjacent coils.

In particular, the use of Helmholtz coils allows the interference of the geomagnetic field to be minimised, thus producing a region with a magnetic field intensity much closer to zero.

Further details regarding the specific structure and functionality of Helmholtz coils are specified in the following article:
- Beiranvand, R. Analyzing the uniformity of the generated magnetic field by a practical one-dimensional Helmholtz coils system. Rev. Sci. Instrum. 2013, 84, 075109.

From an operational viewpoint, the Helmholtz coils are arranged coaxially along a main horizontal axis that extends along a plane in which the bed lies 2b.

In this manner, the bed 2b can be inserted or be insertable inside the treatment volume "T" by sliding the same along the main axis.

In other words, the Helmholtz coils define a substantially cylindrical treatment volume "T" in which the bed can be placed in a fixed or removable manner.

For example, the bed can be movable on horizontal guides allowing it to be slid into and out of the treatment volume "T".

In this context, the device 1 preferably further comprises a compensation coil, which can likewise be made in the form of a Helmholtz coil, configured to compensate for the vertical component of the geomagnetic field.

In particular, the compensation coil is disposed perpendicularly relative to the other coils of the device 1, the Helmholtz coils thus usually being aligned with their central axis disposed horizontally, while the compensation coil is positioned in such a way as to have a central axis disposed vertically.

During use, it can also prove advantageous to position the bed 2b according to a north-south alignment that further enables the elimination of interference due to the horizontal component of the geomagnetic field.

In accordance with a further possible embodiment, the support 2b comprises a seat for the user, on which the latter can sit during use of the device 1.

In this context, the plurality of coils 2a comprises a plurality of Merritt coils; preferably it comprises three Merritt coils.

Further details regarding the specific structure and functionality of Merritt coils are indicated in the following article:
- [80] Merritt R, Purcell C and G Stroink (USA 1983). Uniform magnetic field produced by three, four, and five square coils. Review of Scientific Instruments 1983 March, 54:879; Epub Nov 4th 1998; DOI:10.1063/1.1137480.

In particular, the Merritt coils are arranged coaxially along a main axis, preferably vertical; i.e. the Merritt coils are placed on top of one another so as to define a stack of coils extending vertically.

The seat is placed inside the treatment volume "T", i.e. inside the space delimited by the Merritt coils.

In accordance with a further possible embodiment, the exposure system 2 comprises a booth for the user, inside which the latter can position him- or herself during use of the device 1.

A bed or a seat useful for supporting the user during operation of the device 1 can also be introduced inside the booth.

In this context, the plurality of coils 2a comprises a plurality of Cao coils.

In particular, Cao coils represent a specific embodiment of Merritt coils, whose specific structure and functionality are indicated in the following article:
[81] Qinjie Cao, Donghua Pan, Ji Li, Yinxi Jin, Zhiyin Sun, Shengxin Lin, Guijie Yang and Liyi Li (CHN 2018). Optimization of a Coil System for Generating Uniform Magnetic Fields inside a Cubic Magnetic Shield. Energies 2018, 11, 608

In general, and irrespective of the specific structural conformation of the exposure system 2, the device 1 comprises an impedance meter 4 configured to measure a specific impedance value of a user during use of the device 1, i.e. when the user is inside the treatment volume "T" and is exposed to the electromagnetic field generated by the coils 2a in accordance with the formula (1).

The impedance meter 4 is configured to record a specific impedance signal "I" at the user's body when it is exposed to the electromagnetic field, at an alternating current having the same frequency as is administered by the exposure system 2.

As will be explained in greater depth below, the specific impedance signal "I" will be used by the signal generator 3 to control the operation of the coils 2a.

The device 1 further comprises at least one gaussmeter 4 configured to measure an intensity value of a spatial distribution of the magnetostatic or magnetoquasistatic field with a reading of components in three dimensions, in particular inside the treatment volume "T", and to generate a reference signal "R" representative of that intensity value.

In particular, the gaussmeter 5 is capable of providing the three spatial coordinates of the geomagnetic field in the treatment volume "T" and is connected or connectible to a circuit that calculates the mean and variance of the reference signal "R" over time.

In this manner it is possible to take into account, in a particularly precise and accurate manner, the actual real value of the magnetostatic field in the place and at the moment in which the device 1 is activated.

In order to better understand the significance of this innovation compared to what is offered by the prior art, let us consider the formula that allows a determination of the ion cyclotron resonance frequency, once the static field is known, for the ion cyclotron resonance: 2πv = qB0/m, where v is the desired frequency and B₀ is the modulus of the static field parallel to the variable field.

Let us consider the divalent calcium ion. If we assume B₀=47.8 mT - the value of the geomagnetic field measured by the Observatory of Castel Telesino (TN) on 29/12/2019 - the ion cyclotron resonance frequency of the calcium would be equal to 36.37 Hz.

However, by measuring the field with a reading of the components thereof in the three direction it is possible to take account of the magnetic inclination I₀ in order to determine the real, actual horizontal component, which will be coupled to the variable magnetic field that will be administered, thus B₀=47.8*cos(62.38°), leading to an ion cyclotron resonance frequency of the calcium equal to 16.86 Hz.

More in general, the contribution to the determination of the correct operating frequency of the device 1 given by the geomagnetic field is calculated on the basis of measurements gathered from the impedance meter applying the formula 2πv = (qB0/m) sinI₀ if the variable magnetic field with a frequency v is in the direction of the zenith (vertical) of the measuring point and 2πv = (qB0/m) sinI₀ if the variable magnetic field with a frequency v is tangent to the Earth's surface (horizontal) in the measuring point; where, as indicated above, I₀ is the magnetic inclination in the point where the device 1 is used.

In other words, the reference signal is generated taking into account not only the intensity of the magnetostatic field, in particular of the geomagnetic field, but also the specific magnetic inclination thereof, thus making it possible to obtain a precise measurement that leads to the determination of the correct ion cyclotron resonance frequency.

As will be explained in greater depth below, the reference signal "R" will also be used by the signal generator 3 to control the operation of the coils 2a.

Preferably, the at least one gaussmeter 5 is a precision gaussmeter, that is, it has a high sensitivity, preferably equal to or less than 25 nanoTesla (nT), i.e. it is capable of identifying and discriminating variations in the magnetic field equal to or less than 25 nT.

The signal generator 3, on the other hand, is configured to control the plurality of coils 2a, thereby enabling one or more characteristics of the electromagnetic field to be modulated on the basis of the specific impedance signal "I" and/or the reference signal "R".

In other words, the signal generator receives the specific impedance signal "I" from the impedance meter 4 and the reference signal "R" from the at least one gaussmeter 5 and on the basis of those signals controls and governs the operation of the coils 2a so as to vary the electromagnetic field generated by the latter.

In particular, the signal generator 3 is configured to generate an operating signal "C", which can be either a single magnetic signal at an ion cyclotron resonance frequency for stimulating the formation of a current of ions in tune with the frequency itself; or a double signal, the same one with the addition of a cyclotron frequency of one of the hydroxonium hydrates, in order to increase the conductivity of the intracellular water or a carrier radio signal having a frequency of between 100 and 300 kHz and which can preferably have a sinusoidal or triangular or square or sawtooth waveform, with at least two modulating waves with distinct ion cyclotron frequencies, by means of which it is possible to control the plurality of coils 2a.

Advantageously, the signal generator 3 can be further configured to generate a pre-exposure signal suitable for controlling the operation of the coils 2a at the ion cyclotron resonance frequency of hydroxonium and/or of one of the hydrates thereof (for example the Zundel cation or an Eigencation).

The device 1 can thus be operated under two regimes: a regime controlled by the operating signal "C" and a regime controlled by the pre-exposure signal.

By controlling the operation of the device 1 in a first moment by means of the pre-exposure signal it is possible both to prolong the impact of the Zhadin effect on the ions and protonate any water that may be present in the tissue/sample on which the device 1 is being used, so that the signal/noise ratio of the ionic currents induced by the device 1 itself will be improved when subsequently controlled with the operating signal "C", thus enhancing the overall performance.

The signal generator 3 can also be configured to generate an operating signal "C" that comprises an amplitude-modulated carrier wave and has a frequency comprised between 100 and 300 kHz, by means of which it is possible to modulate the electromagnetic field by controlling the plurality of coils 2a.

In particular, a greater biological effectiveness has been observed in exposure to a low- or very low-frequency amplitude-modulated carrier wave rather than directly to low-frequency waves, as discussed for example in the following publications:
- Bawin SM, Adey WR, Sabbot IM (USA 1978). Ionic factors in release of 45Ca2+ from chicken cerebral tissue by electromagnetic fields. PNAS 1978 Dec;75(12):6314-8;
- Lyle DB, Schechter P, Adey WR, Lundak RL. Suppression of T-lymphocyte cytotoxicity following exposure to sinusoidally amplitude-modulated fields. Bioelectromagnetics. 1983;4(3):281-92;
- Blackman CF, Benane SG, House DE, Joines WT (USA 1985). Effects of ELF (1-120 Hz) and modulated (50 Hz) RF fields on the efflux of calcium ions from brain tissue in vitro. Bioelectromagnetics. 1985;6(1):1-11 ;
- Vignati M, Giuliani L. radiofrequency near high voltage power lines. Environ Health Persp 105(S6):1535.

From an operational viewpoint, the operating signal "C" is generated as a function at least of the reference signal "R" and the specific impedance signal "I" so as to reproduce, in particular, the frequencies at which the user has shown a higher relative specific impedance (i.e. in relation to a specific physiological impedance value), or variation in specific impedance.

This operation is managed by the feedback circuit, which records the variations in specific impedance at the user's body, both of the resistive component and of the reactive one and their relationship, thereby determining, in the event of a variation in the latter parameters beyond certain thresholds that can be programmed, a trigger signal for the signal generator, for the delivery of the magnetic field at the frequency that has brought about the parametric variation beyond the threshold, for a time that is likewise programmable.

In other words, the device 1 has a closed-loop control system in which the spatial distribution of the electromagnetic field inside the treatment volume "T" and the specific impedance of the user are fed back and used to vary the operating signal "C" so as to promptly adapt the coils 2a to generate an electromagnetic field such as to maintain and maximise the efficiency of the ion cyclotron resonance effect.

In particular, the operating signal "C" is varied in such a way as to maintain the device 1 in an operating regime in which the specific impedance signal "I" is maximised.

The determination of the frequency at which the user shows the greatest relative impedance can be made during a preliminary calibration in a global manner, with reference solely to a continuous test exposure current; or local, with a scan that subjects the patient to brief exposures at every selectable frequency, so brief as to cause negligible disturbance, for example in the order of a second.

In other words, it is possible to generate a continuous operating signal "C" to measure the user's response to exposure to the electromagnetic field generated by the coils 2a in general terms, or else perform a progressive scan, exposing the user to all the frequencies that can be generated by the signal generator 3 and accurately mapping the impedance value manifested by the user at every single frequency used.

In this manner, during use of the device 1, the impedance meter 4 will generate the specific impedance signal "I" and the signal generator 3 will compare the new measurements of specific impedance with the values measured in a global or local manner during calibration of the device 1.

In the former case, the comparison will be direct and if for one frequency the ratio between the new measurement and the old one is identical, disregarding the systemic and random errors of the measurement, the patient will be exposed to that frequency for a time and at an intensity defined by the therapist and in any case sufficient to obtain on the user all the beneficial effects deriving from the ion resonance effect.

Ordinarily, exposure is maintained at about ten minutes or a thousand seconds; the intensity will ordinarily be in order of tens and hundreds of micro Tesla or nano Tesla.

The device 1 thus makes it possible to have a real-time control of the effectiveness of the therapeutic action thanks to the continuous readings of the impedance values and spatial distribution of the electromagnetic field generated respectively by the impedance meter and the at least one gaussmeter, further enabling them to be compared with the calibration values in order to determine the operating regime at which the user manifests the maximum response.

The device 1 can further comprise a plurality of sensors configured to detect a respective plurality of physiological parameters and generate a physiological signal representative of at least one of those physiological parameters.

By way of non-limiting example, the plurality of sensors comprises at least one of: a blood pressure sensor, a blood oxygenation sensor and a blood glucose detector.

Advantageously, the signal generator 3 is further configured to control the plurality of coils 2a by modulating one or more characteristics of the electromagnetic field as a function also of the physiological signal considered individually or evaluated in combination with the reference signal "R" and specific impedance signal "I".

In particular, the physiological signal could detect a condition of excessive stress of the user during the use of the device 1 and thus bring about an immediate interruption of the procedure (for example by blocking the generation of the operating signal "C") or generate an error/alert signal of an audible and/or optical type to alert an operator of the occurrence of a situation of discomfort or potential danger to the user's health.

Furthermore, the device 1 can comprise at least one protective casing 6 disposed around the plurality of coils 2a and cooperating therewith to define the treatment volume "T".

In other words, the protective casing 6 can define a physical wall for the treatment volume "T" which serves to cover in particular the portions of the wall of the treatment volume "T" left free by the coils 2a.

The protective casing 6 is configured to shield the treatment volume "T" from the geomagnetic field, thus preventing the latter from disturbing the electromagnetic field and enabling it to be controlled more precisely and efficiently.

In particular, the protective casing can comprise and/or be made of a metallic glass material.

The term metallic glass means a solid metal, usually an alloy, with an atomic structure that is amorphous, hence not crystalline.

In particular, according to a preferred embodiment, the metallic glass comprises a ternary metallic glass alloy based on iron or nickel added to boron in vitreous form and added to yttrium, for example according to the formula Y₄Fe₇₅B₂₁.

The alloy identified above is characterised by a coercivity of around 40 A/m and a specific electric conductivity greater than 1/90. In accordance with possible alternative embodiments, the yttrium can be replaced with an element that has an atomic size of not less than 130% of the size of iron and whose percentage in the alloy is no less than 3% or greater than 10%. The percentage of boron can also vary, between 18 and 27%.

As indicated above, the iron can be replaced at least partially by nickel or, according to possible alternatives that will be discussed in greater detail below, also by structures comprising palladium and/or zirconium in order to make the protective casing 6 from metallic glass material.

In general, the protective casing 6 can be made according to the parametric formula:

Y₃₊ₐFe_{79-a-b-c}Ni_{b}B_{18+c}

wherein the parameters indicated can take on the following values:
- a is comprised between 0 and 3 in atomic percentage;
- b is equal to 0 or 67 in atomic percentage;
- c is comprised between 0 and 9 in atomic percentage.

If a=3, b=67, c=9 the iron is completely replaced in the alloy by nickel.

A greater percentage of boron renders the alloy glassier, whereas a greater percentage of yttrium makes it possible to compensate for the increased molecular weight of the alloy due to the substitution of nickel for iron.

The specific conductivity of the alloy as parametrised above favours the effect of shielding the geomagnetic field.

The use of a protective casing 6 made of a metallic glass material has the further advantage of reducing the risk of occurrence of a feeling of claustrophobia in the user, since it is a material that is transparent to visible light.

Alternatively or additionally, the protective casing 6 can be made of and/or comprise a protective foil (preferably two protective foils, one on top of the other) made of mu-metal.

In accordance with a preferred embodiment, the mu-metal comprises Cu₅Ni₇₇Fe₁₆Cr₂ or Co₂₅₈.

By way of example, a 0.35 mm double layer of permalloy (Ni₅₀₊ₓFe₅₀₋ₓ) or mu-metal would induce, in the case of an industrial electromagnetic field (50/60 Hz), a core loss (substitute for the specific absorption rate for radiofrequencies) of up to 0.2 mW/Kg, i.e. 0.2 µW/g.

Compared to the SAR limit introduced in Europe for partial exposure in the head area, namely, 2 µW/g (also adopted in China; in the USA and Japan it is 20% lower), it would be 10,000 times lower.

Since the performances of such alloys are even better with reference to radiofrequencies and a static magnetic field, this shielding represents the ideal solution for enabling ion cyclotron resonance treatments, given that, in the absence of a geomagnetic field, the system can administer a known static magnetic field with an error of less than a nanoTesla and consequently alternating magnetic fields, including Schumann waves, with equally precise frequencies.

In general it is worth observing that it is advantageously possible also to subtract the self-induced component of the coil from the intensity of the static field component in order to obtain an even more precise measurement.

Furthermore, the device 1 can further comprise a lighting system, for example an LED lighting system.

The lighting system is preferably powered by direct current in order not to cause electromagnetic interference.

In particular, the working frequency of the device 1 is determined also taking into account the static magnetic field induced by the lighting system. Advantageously, the use of the present device 1 permits important applications in the treatment of eukaryotic and prokaryotic cells (and viruses) endowed with pseudovilli extruded above the cell membrane (envelope), with the partial destructuring of the cellular cytoskeleton, due to electromagnetic resonance of actin at 50 Hz, which causes the collapse of pseudomicrovilli within the cytoskeleton.

For example, in the case of the coronavirus the mechanism of infection has been identified as the spike protein (S), consisting of two domains (S1-S2), as in SARS1-CoVid19, and its binding function resides in the S1 domain of the spike protein itself, whereas the molecule that lyses the membrane of the host cell and permits the penetration of the virus resides in the S2 domain.

In SARS2-Covid19 the S1 domain is activated by the enzyme furin, as by the ACE2 receptor of the host cell, a receptor analogous to the ACE receptor of SARS1-CoVid19.

In particular, the spike protein rests, outside the viral envelope, on an actin-like filament that could be made to collapse by exposure to a frequency of 50 Hz or higher, within 100 Hz, with an amplitude in the order of a milliTesla, as the actin filaments of eukaryotic cells collapse under such exposure. See, among many others, S Rieti, V Manni, A Lisi, L Giuliani, et al. SNOM and AFM microscopy techniques to study the effect of non-ionizing radiation on the morphological and biochemical properties of human keratinocytes cell line (HaCaT). J Microsc 213: Pt 1. 20-28 2004 Jan.

In such a case the spike proteins of the coronavirus would all collapse together, ending up in the viral envelope (the physical interactions of sufficient amplitude affect the whole target population at the same instant); and would no longer be able to bind the furin or ACE2 of any host cell.

The effect would have a duration of about one hour and would in any case persist throughout the whole time of exposure, which could be continuous, since exposure to an industrial magnetic field at a few milliTesla is comparable to the exposure limit for workers valid in Italy and other European countries and the possible use of higher exposure values would nonetheless, for the purposes of the present invention, fall within the upper limits (6 mT at the industrial frequency), for the compliance with which earthing of the user's bed or seat and the equipotential bonding thereof would be sufficient.

The therapy implementable by means of the present device 1 aimed at destructuring the pseudo actin filaments of the virus would be effective in particular in the initial stage of infection, i.e. the stage of viral attack.

Any contraindication due to a lower mobility of B lymphocytes could be modulated with an exposure aimed at target organs, also through an exposure probe, in cases of intubation, which only marginally involves circulating blood.

And on the other hand, if the exposure should favour the release of angiotensin, that could contribute to dampening the inflammatory effect of the cytokine storm caused by the virus and would be synergistic with treatment with heparin, for which the therapeutic treatment at high doses has been authorised, on an experimental basis, by the Italian Medicines Agency (AIFA).

The treatment of CoViD19, as well as of SARS, at a severe and critical stage could be supported by exposure to ELF/SLF, given its inhibitory action on interleukin 6, which presumably causes the cytokine storm triggered by the coronavirus.

The same ELF/SLF treatment can be adopted with other coronaviruses, the influenza virus, with picoRNA viruses in general and in diseases caused by viruses that carry binding proteins on extruded filaments of the envelope.

In particular, the two parts S1 and S2 of the spike protein S are mutually mobile and establish between them a potential difference of up to 0.5 V in the binding phase. See, among many others, Cho Y-K, Y-w, Frank M et al. Structure, Dynamics, Receptor Binding, and Antibody Binding of the Fully Glycosylated Full-Length SARS-CoV-2 Spike Protein in a Viral Membrane. J Chem Theory and Comp 2021 17(4):2479-87, doi: 10.1021/acs.jctc.0c01144.

The frequencies between 100 and 200 kHz (indicated as preferential for the device described herein) are capable of altering the charge distribution of the S1 and S2 complexes of the spike protein, preventing the 'jaw-like' functioning whereby the spike protein attaches to the ACE2 receptor with the S1 complex while it lyses the host cell membrane by closing the S2 complex over the attachment point. Similarly, it has been shown that in the electromagnetic treatment of brain tumours long radio waves interfere with the arrangement of homologous charges that form along the parallels of the mitotic spindle of tumour cells during reproduction; as said homologous charges become denser, the spindle dilates until mitosis is reached.

In the same manner, the frequencies indicated would alter the distribution of charges between the SI-S2 subsets of the spike protein, preventing the functioning of the two subsets, above all of S2 in the lysis of the host cell membrane in order to pour viral material inside it.

The device 1 in fact makes it possible to apply a mechanism interfering with the virus SARS-2CoV-19 and to couple, with that basic mechanism, the dual ion cyclotron mechanism due to the double modulators that the device enables control of in the complex of coils 2a.

Advantageously, the present invention achieves the proposed objects, overcoming the aforementioned drawbacks of the prior art by providing the user with a device for ion cyclotron resonance that is particularly versatile and capable of operating under every condition and responding rapidly and efficiently to the conditions of the user during the treatment.

The invention is defined by the appended claims.

## Claims

1. A device for geomagnetic ion cyclotron resonance feedback-controlled by a variation in specific body impedance, comprising:
- an exposure system (2) comprising a plurality of coils (2a) having respective inner surfaces defining a treatment volume (T) adapted to at least partially accommodate a user and configured to administer an electromagnetic field in said treatment volume (T);
- an impedance meter (4) configured to measure a specific impedance value of a user when exposed to said electromagnetic field inside the treatment volume (T) and to generate a specific impedance signal (I) determined by an alternating current having the same frequency as the electromagnetic field administered by the exposure system (2), recording the corresponding impedance value specific to the user's body;
- at least one gaussmeter (5) configured to measure an intensity value of a spatial distribution of the magnetostatic or magnetoquasistatic field with a reading of components in three dimensions inside the treatment volume (T) and to generate a reference signal (R) representative of said intensity value calculated as a function of a magnetic inclination of said magnetostatic or magnetoquasistatic field;
- a signal generator (3) configured to control said plurality of coils (2a) by modulating one or more characteristics of the electromagnetic field by means of an operating signal (C) having a frequency comprised between 100 kHz and 300 kHz generated as a function, simultaneously, of the specific impedance signal (I) and the reference signal (R);
- a feedback circuit configured to record variations in impedance specific to the user's body, both in a resistive component and in a reactive component, as well as in a ratio between the reactive component and the resistive component, determining, in the event of an exceeding of the relative specific impedance or a variation beyond a predefined threshold of said specific impedance, a trigger signal for the signal generator configured to activate, for a predefined time interval, a delivery of the magnetic field at the frequency which determined said variation.

2. The device according to claim 1, comprising a plurality of sensors adapted to detect a respective plurality of physiological parameters and to generate a physiological signal representative of at least one of said physiological parameters, said plurality of sensors preferably comprising at least one of: a blood pressure sensor, a blood oxygenation sensor and a blood glucose detector.

3. The device according to claim 2, wherein the signal generator (3) is further configured to control said plurality of coils (2a) by modulating one or more characteristics of the electromagnetic field as a function also of the physiological signal and of the specific impedance signal (I).

4. The device according to any one of the preceding claims, wherein the signal generator (3) is configured to generate the - operating signal (C) having an amplitude-modulated sinusoidal or triangular or square or sawtooth waveform, with at least two modulating waves with distinct ion cyclotron frequencies controlling the plurality of coils (2a) by means of said operating signal (C).

5. The device according to any one of the preceding claims, wherein the at least one gaussmeter (5) is a precision gaussmeter (5) having a sensitivity equal to or less than 25 nT.

6. The device according to any one of the preceding claims, wherein the exposure system (2) comprises a bed for the user and a plurality of Helmholtz coils (2a), preferably four Helmholtz coils (2a), arranged coaxially along a main horizontal axis, said bed being inserted or insertable in the treatment volume (T) along the main axis.

7. The device according to any one of the preceding claims 1-5, wherein the exposure system (2) comprises a seat for the user and a plurality of Merritt coils (2a), preferably three Merritt coils (2a), arranged coaxially along a main axis, preferably vertical, said seat being inserted in the treatment volume (T).

8. The device according to any one of the preceding claims 1-5, wherein the exposure system (2) comprises a booth for the user and a plurality of Cao coils (2a) coupled to the booth so as to define the treatment volume (T) in cooperation with said booth.

9. The device according to any one of the preceding claims, comprising at least one protective casing (6) disposed around the plurality of coils (2a) and cooperating to define the treatment volume (T), said protective casing (6) being configured to shield the treatment volume (T) from a geomagnetic field.

10. The device according to claim 9, wherein the protective casing (6) comprises a metallic glass material, said metallic glass material preferably comprising Yr3+aFe39+a-b-cNi40+bB18+c, wherein:
- a is comprised between 0 and 3 in atomic percentage;
- b is equal to 0 or 67 in atomic percentage;
- c is comprised between 0 and 9 in atomic percentage.

11. The device according to claim 9 or 10, wherein the protective casing (6) comprises at least one protective foil, said protective foil comprising a mu-metal, said mu-metal preferably comprising Cu5Ni77Fe16Cr2 or Co258.

12. The device according to any one of the preceding claims, comprising an LED lighting system, said lighting system preferably being configured to be powered with direct current.

## Patentansprüche

1. Vorrichtung für geomagnetische Ionenzyklotronresonanz, die durch eine Änderung der spezifischen Körperimpedanz rückkopplungsgesteuert wird, umfassend:
- ein Belichtungssystem (2), das eine Vielzahl von Spulen (2a) mit jeweiligen Innenflächen umfasst, die ein Behandlungsvolumen (T) definieren, das dazu ausgestaltet ist, einen Benutzer oder eine Benutzerin zumindest teilweise aufzunehmen, und dazu ausgelegt ist, ein elektromagnetisches Feld in dem Behandlungsvolumen (T) zu verabreichen;
- einen Impedanzmesser (4), der ausgelegt ist, um einen spezifischen Impedanzwert eines Benutzers oder einer Benutzerin zu messen, wenn er dem elektromagnetischen Feld innerhalb des Behandlungsvolumens (T) ausgesetzt ist, und um ein spezifisches Impedanzsignal (I) zu erzeugen, das durch einen Wechselstrom bestimmt wird, der die gleiche Frequenz wie das elektromagnetische Feld aufweist, das durch das Belichtungssystem (2) verabreicht wird, wobei der entsprechende Impedanzwert, der für den Körper des Benutzers oder der Benutzerin spezifisch ist, aufgezeichnet wird;
- mindestens ein Gausmeter (5), das ausgelegt ist, um einen Intensitätswert einer räumlichen Verteilung des magnetostatischen oder magnetoquasistatischen Feldes mit einem Ablesen von Komponenten in drei Dimensionen innerhalb des Behandlungsvolumens (T) zu messen und um ein Referenzsignal (R) zu erzeugen, das für den Intensitätswert repräsentativ ist, der als Funktion einer magnetischen Neigung des magnetostatischen oder magnetoquasistatischen Feldes berechnet wird;
- einen Signalgenerator (3), der ausgelegt ist, um die Vielzahl von Spulen (2a) durch Modulieren einer oder mehrerer Eigenschaften des elektromagnetischen Feldes mittels eines Betriebssignals (C) mit einer Frequenz zwischen 100 kHz und 300 kHz zu steuern, das gleichzeitig als Funktion des spezifischen Impedanzsignals (I) und des Referenzsignals (R) erzeugt wird;
- eine Rückkopplungsschaltung, die dazu ausgelegt ist, Änderungen der Impedanz, die für den Körper des Benutzers oder der Benutzerin spezifisch sind, sowohl in einer Widerstandskomponente als auch in einer Reaktivkomponente sowie in einem Verhältnis zwischen der Reaktivkomponente und der Widerstandskomponente aufzuzeichnen, wobei es bewirkt wird, dass im Falle eines Überschreitens der relativen spezifischen Impedanz oder einer Änderung über einen vordefinierten Schwellenwert der spezifischen Impedanz ein Triggersignal für den Signalgenerator dazu ausgelegt ist, für ein vordefiniertes Zeitintervall eine Abgabe des Magnetfelds mit der Frequenz zu aktivieren, die die Änderung bewirkt hat.

2. Vorrichtung nach Anspruch 1, umfassend eine Vielzahl von Sensoren, die dazu ausgestaltet sind, eine jeweilige Vielzahl von physiologischen Parametern zu erfassen und ein physiologisches Signal zu erzeugen, das für mindestens einen der physiologischen Parameter repräsentativ ist, wobei die Vielzahl von Sensoren vorzugsweise mindestens eines von Folgendem umfasst: einen Blutdrucksensor, einen Blutoxygenierungssensor und einen Blutglucosedetektor.

3. Vorrichtung nach Anspruch 2, wobei der Signalgenerator (3) ferner dazu ausgelegt ist, die Vielzahl von Spulen (2a) durch Modulieren einer oder mehrerer Eigenschaften des elektromagnetischen Feldes als eine Funktion auch des physiologischen Signals und des spezifischen Impedanzsignals (I) zu steuern.

4. Vorrichtung nach einem der vorhergehenden Ansprüche, wobei der Signalgenerator (3) ausgelegt ist, um das Betriebssignal (C) mit einer amplitudenmodulierten sinusförmigen oder dreieckförmigen oder quadratischen oder sägezahnförmigen Wellenform zu erzeugen, wobei mindestens zwei modulierende Wellen mit unterschiedlichen Ionenzyklotronfrequenzen die Vielzahl von Spulen (2a) mittels des Betriebssignals (C) steuern.

5. Vorrichtung nach einem der vorhergehenden Ansprüche, wobei das mindestens eine Gaussmeter (5) ein Präzisions-Gaussmeter (5) mit einer Empfindlichkeit von gleich oder weniger als 25 nT ist.

6. Vorrichtung nach einem der vorhergehenden Ansprüche, wobei das Belichtungssystem (2) ein Bett für den Benutzer oder die Benutzerin und eine Vielzahl von Helmholtz-Spulen (2a), vorzugsweise vier Helmholtz-Spulen (2a), die entlang einer horizontalen Hauptachse koaxial angeordnet sind, umfasst, wobei das Bett entlang der Hauptachse in das Behandlungsvolumen (T) eingesetzt oder einsetzbar ist.

7. Vorrichtung nach einem der vorhergehenden Ansprüche 1-5, wobei das Belichtungssystem (2) einen Sitz für den Benutzer oder die Benutzerin und eine Vielzahl von Merritt-Spulen (2a), vorzugsweise drei Merritt-Spulen (2a), umfasst, die koaxial entlang einer Hauptachse, vorzugsweise vertikal, angeordnet sind, wobei der Sitz in das Behandlungsvolumen (T) eingesetzt ist.

8. Vorrichtung nach einem der vorhergehenden Ansprüche 1-5, wobei das Belichtungssystem (2) eine Kabine für den Benutzer oder die Benutzerin und eine Vielzahl von Cao-Spulen (2a) umfasst, die mit der Kabine gekoppelt sind, um das Behandlungsvolumen (T) in Zusammenarbeit mit der Kabine zu definieren.

9. Vorrichtung nach einem der vorhergehenden Ansprüche, umfassend mindestens ein Schutzgehäuse (6), das um die Vielzahl von Spulen (2a) herum angeordnet ist und zusammenwirkt, um das Behandlungsvolumen (T) zu definieren, wobei das Schutzgehäuse (6) ausgelegt ist, um das Behandlungsvolumen (T) von einem geomagnetischen Feld abzuschirmen.

10. Vorrichtung nach Anspruch 9, wobei das Schutzgehäuse (6) ein metallisches Glasmaterial umfasst, wobei das metallische Glasmaterial vorzugsweise Yr3+aFe39+a-b-cNi40+bB18+c umfasst, wobei:
- a zwischen 0 und 3 in Atomprozent liegt;
- b gleich 0 oder 67 in Atomprozent ist;
- c zwischen 0 und 9 in Atomprozent liegt.

11. Vorrichtung nach Anspruch 9 oder 10, wobei das Schutzgehäuse (6) mindestens eine Schutzfolie umfasst, wobei die Schutzfolie ein Mu-Metall umfasst, wobei das Mu-Metall vorzugsweise Cu5Ni77Fe16Cr2 oder Co258 umfasst.

12. Vorrichtung nach einem der vorhergehenden Ansprüche, umfassend ein LED-Beleuchtungssystem, wobei das Beleuchtungssystem vorzugsweise dazu ausgelegt ist, mit Gleichstrom versorgt zu werden.

## Revendications

1. Dispositif pour la résonance cyclotron ionique géomagnétique commandé par rétroaction par une variation d'impédance corporelle spécifique, comprenant :
- un système d'exposition (2) comprenant une pluralité de bobines (2a) comportant des surfaces intérieures respectives définissant un volume de traitement (T) adapté pour loger au moins partiellement un utilisateur et configuré pour administrer un champ électromagnétique dans ledit volume de traitement (T) ;
- un impédancemètre (4) configuré pour mesurer une valeur d'impédance spécifique d'un utilisateur lorsqu'il est exposé audit champ électromagnétique à l'intérieur du volume de traitement (T) et pour générer un signal d'impédance spécifique (I) déterminé par un courant alternatif ayant la même fréquence que le champ électromagnétique administré par le système d'exposition (2), enregistrer la valeur d'impédance correspondante spécifique au corps de l'utilisateur ;
- au moins un gaussmètre (5) configuré pour mesurer une valeur d'intensité d'une distribution spatiale du champ magnétostatique ou magnétoquasi-statique avec une lecture de composants en trois dimensions à l'intérieur du volume de traitement (T) et pour générer un signal de référence (R) représentatif de ladite valeur d'intensité calculée en fonction d'une inclinaison magnétique dudit champ magnétostatique ou magnétoquasi-statique ;
- un générateur de signaux (3) configuré pour commander ladite pluralité de bobines (2a) en modulant une ou plusieurs caractéristiques du champ électromagnétique au moyen d'un signal de fonctionnement (C) ayant une fréquence comprise entre 100 et 300 kHz générée en fonction, simultanément, du signal d'impédance spécifique (I) et du signal de référence (R) ;
- un circuit de rétroaction configuré pour enregistrer des variations d'impédance spécifiques au corps de l'utilisateur, à la fois dans un composant résistif et dans un composant réactif, ainsi que dans un rapport entre le composant réactif et le composant résistif, déterminant, en cas de dépassement de l'impédance spécifique relative ou d'une variation au-delà d'un seuil prédéfini de ladite impédance spécifique, un signal de déclenchement pour le générateur de signal configuré pour activer, pendant un intervalle de temps prédéfini, une distribution du champ magnétique à la fréquence qui a déterminé ladite variation.

2. Dispositif selon la revendication 1, comprenant une pluralité de capteurs adaptés pour détecter une pluralité respective de paramètres physiologiques et pour générer un signal physiologique représentatif d'au moins un desdits paramètres physiologiques, ladite pluralité de capteurs comprenant de préférence au moins un des éléments suivants : un capteur de pression sanguine, un capteur d'oxygénation sanguine et un détecteur de glycémie.

3. Dispositif selon la revendication 2, dans lequel le générateur de signaux (3) est en outre configuré pour commander ladite pluralité de bobines (2a) en modulant une ou plusieurs caractéristiques du champ électromagnétique en fonction également du signal physiologique et du signal d'impédance spécifique (I).

4. Dispositif selon l'une quelconque des revendications précédentes, dans lequel le générateur de signaux (3) est configuré pour générer le signal de fonctionnement (C) ayant une forme d'onde sinusoïdale ou triangulaire ou carrée ou en dents de scie modulée en amplitude, avec au moins deux ondes de modulation ayant des fréquences de cyclotron ioniques distinctes commandant la pluralité de bobines (2a) au moyen dudit signal de fonctionnement (C).

5. Dispositif selon l'une quelconque des revendications précédentes, dans lequel l'au moins un gaussmètre (5) est un gaussmètre (5) de précision ayant une sensibilité égale ou inférieure à 25 nT.

6. Dispositif selon l'une quelconque des revendications précédentes, dans lequel le système d'exposition (2) comprend un lit pour l'utilisateur et une pluralité de bobines de Helmholtz (2a), de préférence quatre bobines de Helmholtz (2a), disposées coaxialement le long d'un axe horizontal principal, ledit lit étant inséré ou insérable dans le volume de traitement (T) le long de l'axe principal.

7. Dispositif selon l'une quelconque des revendications précédentes 1-5, dans lequel le système d'exposition (2) comprend un siège pour l'utilisateur et une pluralité de bobines de Merritt (2a), de préférence trois bobines de Merritt (2a), disposées coaxialement le long d'un axe principal, de préférence vertical, ledit siège étant inséré dans le volume de traitement (T).

8. Dispositif selon l'une quelconque des revendications précédentes 1-5, dans lequel le système d'exposition (2) comprend une cabine pour l'utilisateur et une pluralité de bobines de Cao (2a) couplées à la cabine de manière à définir le volume de traitement (T) en coopération avec ladite cabine.

9. Dispositif selon l'une quelconque des revendications précédentes, comprenant au moins un boîtier de protection (6) disposé autour de la pluralité de bobines (2a) et coopérant pour définir le volume de traitement (T), ledit boîtier de protection (6) étant configuré pour protéger le volume de traitement (T) d'un champ géomagnétique.

10. Dispositif selon la revendication 9, dans lequel le boîtier de protection (6) comprend un matériau en verre métallique, ledit matériau en verre métallique comprenant de préférence Yr3+aFe39+a-b-cNi40+bB18+c, dans lequel :
- a est compris entre 0 et 3 en pourcentage atomique ;
- b est égal à 0 ou 67 en pourcentage atomique ;
- c est compris entre 0 et 9 en pourcentage atomique.

11. Dispositif selon la revendication 9 ou 10, dans lequel le boîtier de protection (6) comprend au moins une feuille de protection, ladite feuille de protection comprenant un métal Mu, ledit métal Mu comprenant de préférence Cu5Ni77Fe16Cr2 ou Co258.

12. Dispositif selon l'une quelconque des revendications précédentes, comprenant un système d'éclairage à LED, ledit système d'éclairage étant de préférence configuré pour être alimenté en courant continu.
